(19) Europäisches Patentamt / European Patent Office / Office européen des brevets

(11) **EP 3 476 918 B1**

(12) **EUROPEAN PATENT SPECIFICATION**

(45) Date of publication and mention
of the grant of the patent:
**17.07.2024 Bulletin 2024/29**

(21) Application number: **18199677.8**

(22) Date of filing: **10.10.2018**

(51) International Patent Classification (IPC):
**C07C 31/18** (2006.01)　　　**C07C 29/132** (2006.01)
**C10G 3/00** (2006.01)

(52) Cooperative Patent Classification (CPC):
(C-Sets available)
**C10G 3/50; C07C 29/132;** Y02P 30/20　　　(Cont.)

(54) **METHOD FOR RECOVERING A DISUSED OIL REFINERY**

VERFAHREN ZUR RÜCKGEWINNUNG EINER STILLGELEGTEN ÖLRAFFINERIE

PROCÉDÉ DE RÉCUPÉRATION D'UNE RAFFINERIE DE PÉTROLE MISE AU REBUT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2017 IT 201700122279**

(43) Date of publication of application:
**01.05.2019 Bulletin 2019/18**

(73) Proprietor: **Water & Soil Remediation S.r.l.**
**46010 Curtatone, Frazione Levata MN (IT)**

(72) Inventor: **PRANDI, Alberto**
**46100 MANTOVA (IT)**

(74) Representative: **Modiano, Micaela Nadia et al**
**Modiano & Partners**
**Via Meravigli, 16**
**20123 Milano (IT)**

(56) References cited:
WO-A1-2014/033762　　　WO-A1-2015/181279
US-A1- 2012 095 274

- JUAN ANTONIO MELERO ET AL: "Biomass as renewable feedstock in standard refinery units. Feasibility, opportunities and challenges", ENERGY & ENVIRONMENTAL SCIENCE, vol. 5, no. 6, 1 January 2012 (2012-01-01), pages 7393, XP055046864, ISSN: 1754-5692, DOI: 10.1039/c2ee21231e
- JUAN CARLOS SERRANO-RUIZ ET AL: "CATALYTIC PRODUCTION OF LIQUID HYDROCARBON TRANSPORTATION FUELS", 31 December 2012 (2012-12-31), XP002715022, ISBN: 978-1-4614-0343-2, Retrieved from the Internet <URL:www.springer.com/978-1-4614-0343-2>
- HUBER G W ET AL: "An overview of aqueous-phase catalytic processes for production of hydrogen and alkanes in a biorefinery", CATALYSIS TODAY, ELSEVIER, AMSTERDAM, NL, vol. 111, no. 1-2, 15 January 2006 (2006-01-15), pages 119 - 132, XP027975836, ISSN: 0920-5861, [retrieved on 20060115]

　　　**(Cont. next page)**

(52) Cooperative Patent Classification (CPC): (Cont.)

C-Sets
**C07C 29/132, C07C 31/18**

**Description**

The present invention relates to a method for recovering a disused oil refinery.

The use is known of processes for hydrogenation and/or hydrogenolysis of carbohydrates, i.e., of chemical compounds formed by atoms of carbon, hydrogen and oxygen, also known as glucides or saccharides, in order to obtain certain products.

For example, hydrogenation of xylose aimed at producing xylitol, with a continuous process, and hydrogenation of glucose to produce sorbitol are known.

Xylitol, like sorbitol, is a sweetener that is less harmful to health than traditional sugars and therefore is becoming increasingly widespread, to the detriment of traditional sweeteners.

In the background art, xylitol is produced by catalytic hydrogenation of xylose, typically performed in a three-phase slurry batch reactor, where the catalyst is kept dispersed in the reaction solution for all the time required to complete the chemical reaction and the water is used as solvent.

In particular, slurry catalysis is suitable for processes of a discontinuous type, known in the jargon as batch processes, in which at the end of the reaction the catalyst is separated physically from the aqueous solution by filtration and/or centrifugation. The discontinuity of the slurry process is a severe limitation, both because it is not adapted to the production of large quantities and because each batch entails unknowns regarding the reproducibility of the operating conditions and consequently regarding the yields and quality of the finished product.

In recent industrial applications, the process with a fixed reactor, for example of the drip type, is becoming widespread and has the great advantage of being able to operate continuously and thus benefit from good productions in terms of both quantity and quality. In this case, the reacting two-phase mixture, composed of an aqueous solution of xylose and hydrogen, once brought to the chosen temperature and pressure conditions, passes through a fixed-bed catalyst, which facilitates the occurrence of the chemical hydrogenation reaction.

Various catalysts for the various chemical reactions, including fixed-bed ones for hydrogenation of xylose aimed at obtaining xylitol, are currently commercially available.

Although the technology of the continuous hydrogenation process by fixed-bed reactor is progressively replacing the batch technology in the field of industrial productions, even today, however, catalysts for the hydrogenation of xylose to xylitol and of other sugars in general are affected by rapid deactivation, often due to occlusion of the pores inside the catalytic medium, and consequent loss of the active centers that are responsible for catalysis.

Partial restoration of the catalytic activity of hydrogenation catalysts can be obtained by scrubbing the reactor with a solvent that is capable of restoring the porosity of the catalyst, but this entails halting the plant and the loss of production capacity.

Moreover, the reactive part of fixed-bed catalytic plants is the most significant portion of the total investment costs for this type of plant, since it is the part that is subjected to the highest temperatures and pressures and therefore requires the most valuable materials with respect to the remaining parts of such plants. Moreover, indeed due to these reasons, in industrial practice there is a tendency not to provide backup reactors.

This is one of the limitations of current plants for the continuous hydrogenation of sugars, in addition to the inherent one of the high cost of a plant built from scratch.

It is also known that as a consequence of the oil crises of the recent decades and of the problem of global warming, a significant and rising overcapacity of oil refineries and petrochemical facilities has occurred, with the closure of many facilities in Italy and Europe; at the same time, the need to replace fossil raw materials with renewable ones, not only in the energy field, is increasingly lively.

When oil and petrochemical facilities are disused, they are often abandoned or even dismantled without reusing the existing infrastructures for other production activities.

In order to overcome these problems, methods for converting conventional refineries into biorefineries have been proposed. For example, WO 2015/181279 A and WO 2014/033762 A disclose revamping a conventional refinery of mineral oils into a biorefinery.

However, according to these methods, existing structures and equipment of oil refineries are converted to perform the biorefining of residual lignocellulosic biomasses and therefore of waste or reject substances in order to obtain mainly biofuels.

The aim of the present invention is to provide a method for recovering a disused oil refinery that is capable of improving the background art in one or more of the aspects indicated above.

Within this aim, an object of the invention is to provide a method for recovering a disused oil refinery that is capable of offering a valid alternative to the simple dismantling of abandoned oil refineries.

Another object of the invention is to provide a method for recovering a disused oil refinery that allows to obtain a plant for the production of carbohydrate-based products.

A further object of the invention is to provide a method for recovering a disused oil refinery that offers a solution to the problem of the high investment costs of plants for continuous hydrogenation and/or hydrolysis of carbohydrates.

Another object of the invention is to provide a method for recovering a disused oil refinery that can be performed easily at sustainable costs.

Furthermore, an object of the present invention is to overcome the drawbacks of the background art in a manner that is alternative to any existing solutions.

This aim, as well as these and other objects which will become better apparent hereinafter, are achieved by a method for recovering a disused oil refinery according to claim 1, optionally provided with one or more of the characteristics of the dependent claims.

Further characteristics and advantages of the invention will become better apparent from the description of some preferred but not exclusive embodiments of the method according to the invention, illustrated by way of nonlimiting example in the accompanying drawings, wherein:

Figure 1 is a schematic view of a typical plant for the catalytic hydrotreatment of oil fractions;
Figure 2 is a schematic view of a hydrogenation plant obtained by means of the method according to the invention.

With reference to the figures, the method according to the invention is applicable to a disused oil refinery or also to a disused petrochemical plant comprising at least one plant for the catalytic hydrotreatment of oil products, for example hydrodesulfurization or hydroisomerization or hydrocracking.

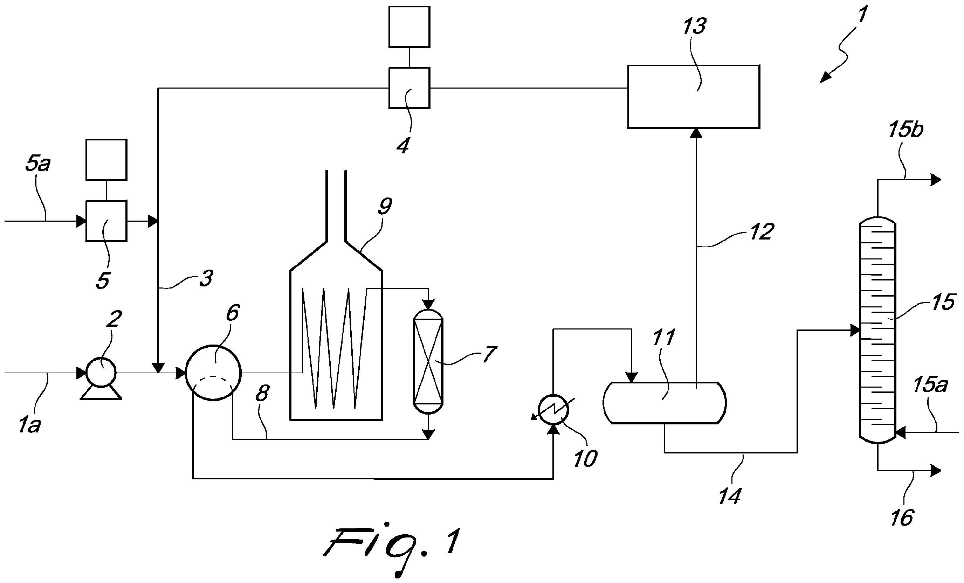

In particular, Figure 1 shows and generally designates with the reference numeral 1 a typical plant for the execution of a process for catalytic hydrotreatment of oil fractions, for example hydrodesulfurization, in which the hydrogen reacts catalytically with the sulfurated organic compounds in order to obtain sulfurated hydrogen, which is then separated from the oil fraction, which is thus desulfurized.

This hydrotreatment process typically occurs on a fixed-bed catalyst contained in a reactor, which is crossed by the reaction mixture at a given temperature and pressure as required by each specific application.

In the example of hydrodesulfurization, the following typical chemical reaction occurs on the active centers of the catalyst:

$$R_1\text{-}S\text{-}R_2 + 2H_2 = R_1H + R_2H + H_2S$$

where $R_1H$ and $R_2H$ are light hydrocarbons which, together with the sulfurated hydrogen $H_2S$, are subsequently released from the desulfurized oil product by means of a stripper, as described hereinafter.

More particularly, with reference to the plant of Figure 1, the load being fed, constituted by a hydrocarbon fraction, is drawn, from an intake line 1a, by means of a supply pump or plant loading pump 2, and receives the addition of the hydrogen required for the subsequent treatments by virtue of hydrogen input means, which commonly comprise a line 3 for feeding hydrogen which is connected in output to the intake line 1a, preferably downstream of the supply pump 2, and in input to a hydrogen recycling compressor 4 and to a makeup compressor 5, which in turn is connected at the intake to a hydrogen input line 5a.

The mixture being fed, composed of the load and the hydrogen, thus compressed, is then introduced in a load/effluent heat exchange unit 6 or preheating unit, in which, by means of one or more heat exchangers, it receives heat from the mixture that flows out of a reactive section.

The mixture being fed then reaches a furnace 9 for further heating to the temperature provided for the catalytic reaction and then enters the reactive section, constituted by a reactor 7, where an adapted catalyst facilitates the reaction of hydrotreatment of some oil compounds, for example the sulfurated compounds with production of sulfurated hydrogen.

The mixture that exits from the reactor 7, by means of a discharge line 8, is partially cooled in the exchange unit 6, in countercurrent with the load being fed, and sometimes further cooled in a cooler 10 before expanding in a gas/liquid separator 11, in which the excess hydrogen is released in the gaseous phase, so that it can be recycled and introduced upstream of the load/effluent exchange unit 6, by means of a return line 12 which is connected to the feed line 3 and by means of the recycling compressor 4, after undergoing scrubbing to remove the unwanted gases in a scrubbing station 13, where for example the sulfurated hydrogen, in the case of hydrodesulfurizations, is scrubbed with an amine compound.

The liquid phase, constituted, in the case being considered, by the desulfurized oil product, flows through a supply line 14 toward a stripping column or stripper 15, which receives heat from a line 15a for the inflow of a heating fluid and frees it from the excess hydrogen and from the light compounds produced in the hydrogenation reaction and, in the case being considered, the sulfurated hydrogen, which exit from the stripping column 15 through an extraction line 15b, before being conveyed to storage by means of an output line 16. The net consumption of hydrogen is integrated in the plant 1 with the aid of the makeup compressor 5.

With respect to the typical plant for catalytic hydrotreatment of oil fractions described above, in industrial practice there are various plant variations that are customized for the individual production situations.

For example, in these variations, gas/liquid separation can occur in multiple stages. In this case, the current that flows out of the reactor 7 and is released from the gas/liquid separator 11 is further cooled and then expanded in a second separator at low pressure, where some low-boiling hydrocarbon compounds are recovered before entering the stripping column 15. Furthermore, other units can be inserted in the plant 1, for example additional heat exchangers and cooling elements, filters, coalescence devices or coalescers, and condensate separators, etc.

In any case, catalytic hydrotreatment plants used in oil refineries substantially follow the basic layout shown in Figure 1 and essentially comprise at least one supply pump 2, heat exchange means, which comprise load/effluent exchangers or a load/effluent heat exchange unit 6, and at least one furnace 9, a reactive section comprising at least one reactor 7, optionally other heat exchangers, such as at least one cooler 10, at least one gas/liquid separator 11, means for restoring and recycling hydrogen after purification, conveniently obtained by means of at least one makeup compressor 5, at least one hydrogen recycling compressor 5 and a scrubbing station 13, and at least one column 15 for stripping the reacted product.

In accordance with the method according to the invention, the hydrotreatment plant provided with this basic layout and derived from a disused refinery is at least partially subjected to reuse in order to obtain a plant 100 for the hydrogenation of xylose, with minimal investment cost.

Conveniently, some components of the plant 1 may be reused in the plant 100 practically as they are.

In any case, provisions are made for replacement of the catalyst in the reactor 7 with a catalyst adapted to facilitate a xylose hydrogenation reaction to obtain xylitol.

More particularly, the components of the hydrotreatment plant 1 derived from the disused refinery which, conveniently, can be reused, according to the proposed method, in order to obtain the plant 100 for the hydrogenation of xylose, can at least comprise: the supply pump 2; the hydrogen input means; the load/effluent heat exchange unit 6; the reactor 7, after replacement of the catalyst; the gas/liquid separator 11; the stripping column 15; the ducts for connection between these components.

It should be pointed out that before their reuse the components of the plant 1 are advantageously subjected to complete cleaning, which is typically performed first with a prolonged passage of water vapor, which allows to move the liquids previously accumulated in their internal spaces and on the bottom of the ducts and furthermore produces the evaporation of the residues that have adhered to the metallic walls. Scrubbing then continues with flushing with water. Finally, all the vessels and the heat exchangers that are present and intended for reuse are opened, inspected and if necessary cleaned with mechanical processes.

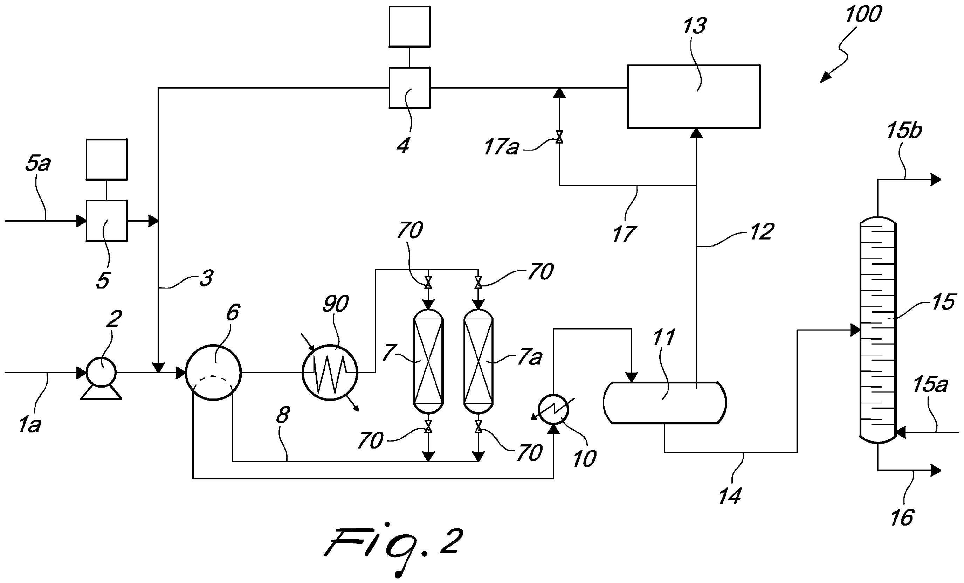

With reference to Figure 2, the path is described hereinafter, by way of example, of a flow of a saccharide in solution through a plant 100 which can be obtained, according to the proposed method, from the reuse of the plant 1, in order to be hydrogenated.

According to the presently claimed invention, the saccharide being loaded is xylose and its hydrogenation product is xylitol.

The saccharide load is provided in input to the intake line 1a, so that it is drawn by the plant loading pump 2 and receives the addition, by means of the feed line 3, of the necessary hydrogen, which is conveniently supplied under pressure by the recycling compressor 4 and by the makeup compressor 5.

The load of the saccharide being fed, with this addition, is then introduced in the load/effluent heat exchange unit 6, in which it receives heat from the discharge line 8.

The load then enters another heat exchanger 90, which can replace the furnace 9 of the plant 1, as explained better hereinafter, for further heating up to the temperature provided for the catalytic reaction, and then enters the reactor 7, where an appropriate catalyst, which as mentioned has taken the place of the one used previously for the plant 1, facilitates the hydrogenation reaction of the xylose. In the case being considered, xylose is hydrogenated, forming xylitol according to the following reaction:

$$C_5H_{10}O_5 + H_2 = C_5H_{12}O_5.$$

The mixture that exits from the reactor 7 through the discharge line 8 is partially cooled in the heat exchange unit 6, in countercurrent with the load in input, and if necessary is further cooled in the cooler 10 before it is left to expand in the gas/liquid separator 11, in which the excess hydrogen is released in the gaseous phase and is recycled at the top of the load/effluent exchange unit 6 by means of the recycling compressor 5 and after removal, performed in the scrubbing station 13, of the unwanted gases, constituted for example by light compounds which are byproducts of hydrogenation.

The liquid phase, which is constituted in the case being considered by the hydrogenated xylose and exits from the gas/liquid separator 11, flows through the supply line 14 toward the stripping column 15, in which it is separated from the excess hydrogen and from light compounds which are byproducts of the reaction, which are evacuated by means of the extraction line 15b.

The net consumption of hydrogen is restored in the plant 100 again with the aid of the makeup compressor 5.

For the reactive section of the plant 100 it is possible to provide an enhancement, with respect to the section of the plant 1, since some catalysts undergo a progressive deactivation over the course of their life due to occlusion of porosities of the medium of the active catalysis centers, which progressively decrease in number, and this requires operations for scrubbing, regeneration and finally replacement of the catalytic mass, halting the plant and losing production.

As a remedy to these drawbacks, the method according to the invention provides for the installation in the plant 100 of at least one additional reactor 7a in addition to the reactor 7 that derives from the plant 1.

The reactors 7 and 7a are arranged in parallel to each other and can be used selectively, by opening/closing flow control valves 70, which are preferably arranged in input and in output to the reactors 7 and 7a, so that one can always be operating, thus keeping the plant 100 active even in case of intervention on the catalytic bed of the other plant. The additional reactor or reactors 7a also can be obtained by reusing other reactors or vessels taken from other disused oil plants or plants previously used for other functions which are in any case oil-related or petrochemical.

It is optionally possible to provide for the installation, in the plant 100, of a line 17 for bypassing the scrubbing station 13 which is controlled by an opening/closure valve 17a.

It is possible to deduce, from what has been described, that oil plants for hydrotreatment that follow the same basic process scheme described in Figure 1, which consists essentially of: supply pumps, load/effluent exchangers, furnace, reactor, other heat exchangers, gas/liquid separation means, such as the gas/liquid separator 10, means for restoring and recycling hydrogen after purification, columns for stripping the reacted product, can, by means of the proposed method, be reused or in any case converted to perform the hydrotreatment of carbohydrates at least in their fundamental components.

More particularly, the method according to the invention provides for reuse at least of the reactive section, but after the removal of the existing catalyst and its replacement with a catalyst that is suitable to facilitate a reaction of hydrogenation of xylose, of the heat exchange means and in particular of the load/effluent heat exchange unit 6, of the gas/liquid separation means, of the hydrogen input means and in particular the compressors 4 and 5, in addition to the possibility to reuse the ducts for the mutual interconnection of the various components of the plant 1, such as the already existing lines 1a, 3, 8, 12, 14, 16, as well as the instruments and the various secondary equipment.

It should be noted that reuse of the gas/liquid separation means is provided even if they are divided into multiple stages.

Furthermore, it is possible to advantageously reuse, in order to obtain the plant 100, also filters, coalescence devices, additional separation means, additional heat exchangers, vacuum systems, if they already exist.

According to the presently claimed method, the furnace 9 that is present in the plant 1 is eliminated and replaced, in the plant 100, with a heat exchanger 90 which is supplied with hot fluid, since xylose require relatively low reaction temperatures for hydrogenation, on the order of slightly above 100°C rather than above 300°C as typically required by hydrotreatments of hydrocarbons.

With the plant 100 thus obtained it thus becomes possible to perform conversions of hot fluids that are frequently present in facilities, in order to bring the mixture to the reaction temperature instead of the furnace 9. In this case, benefits of an environmental kind as well as of an economic kind are achieved, since it is possible to renounce combustion for the heating being considered.

It should also be noted that the operating pressures required to hydrogenate xylose are distinctly lower than the ones previously adopted in oil-related functions; due to this aspect also, there are no hindrances to the operations for reuse of the components of the plant 1 in order to provide the plant 100.

As regards furthermore the corrosiveness of the processes, the conditions in the case of carbohydrates are generally less corrosive than hydrocarbon-related ones, such as to keep the existing metallurgies valid.

It is estimated that the method according to the invention allows to save an investment fraction on the order of 80% with respect to the investment cost required to build a new continuous production plant.

The scope of the invention is defined by the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

**Claims**

1. A method for recovering a disused oil refinery to obtain a plant adapted to produce xylitol from xylose,

   said refinery comprising at least one plant for the catalytic hydrotreatment of oil products (1) provided at least with the following components:

   - at least one supply pump (2);

- means for introducing hydrogen;
- heat exchange means comprising at least one preheating unit and at least one furnace (9);
- at least one reactive section which comprises at least one reactor (7);
- gas/liquid separation means;
- at least one column (15) for stripping the reacted product;

said method being **characterized in that** it provides for a step of reusing at least partly said components in order to obtain a plant for continuous hydrogenation of xylose to xylitol (100), and it provides for:

- removing, in said reactive section, the catalyst that is present and replacing it with a catalyst that is adapted to facilitate xylose hydrogenation reaction to obtain xylitol;
- keeping substantially as they are at least said at least one supply pump (2), said hydrogen input means, said at least one preheating unit, said gas/liquid separation means, and the corresponding mutual interconnection ducts, and removing said furnace (9) and replacing it with a heat exchanger (90) supplied with hot fluid; and
- supplying xylose in input to said supply pump (2).

2. The method according to one or more of the preceding claims, **characterized in that** it provides for the reuse also of said stripping column (15).

3. The method according to one or more of the preceding claims, **characterized in that** it provides for the installation, in said reactive section, of at least one additional reactor (7a) in parallel with said at least one reactor (7) and so that said reactor (7) and said additional reactor (7a) can be used selectively.

4. The method according to one or more of the preceding claims, **characterized in that** said at least one additional reactor (7a) derives from the reuse of an existing reactor or vessel that originates from a disused refinery or was previously used for other functions.

**Patentansprüche**

1. Ein Verfahren zur Rückgewinnung einer stillgelegten Ölraffinerie, um eine Anlage zu erhalten, die ausgebildet ist, um Xylitol aus Xylose zu erzeugen,

wobei die Raffinerie mindestens eine Anlage (1) zum katalytischen Hydrotreating von Ölprodukten umfasst, ausgestattet mit mindestens folgenden Komponenten:

- mindestens einer Versorgungspumpe (2);
- Mitteln zum Einlassen von Wasserstoff;
- Wärmetauschmitteln, die mindestens eine Vorheizeinheit und mindestens einen Ofen (9) umfassen;
- mindestens einem reaktiven Abschnitt, der mindestens einen Reaktor (7) umfasst;
- Gas-Flüssigkeits-Trennmitteln;
- mindestens einer Säule (15) zum Abtreiben des umgesetzten Produkts;

wobei das Verfahren **dadurch gekennzeichnet ist, dass** es einen Schritt zur zumindest partiellen Wiederverwendung der Komponenten vorsieht, um eine Anlage (100) zur kontinuierlichen Hydrierung von Xylose zu Xylitol zu erhalten, und dass es Folgendes bereitstellt:

- das Entfernen, in dem reaktiven Abschnitt, des vorhandenen Katalysators und das Ersetzen desselben durch einen Katalysator, der ausgebildet ist, um die Xylose-Hydrierungsreaktion, um Xylitol zu erhalten, zu erleichtern;
- das Halten der mindestens einen Versorgungspumpe (2), der Wasserstoffeinlassmittel, der mindestens einen Vorheizeinheit, der Gas-Flüssigkeits-Trennmittel und der entsprechenden Leitungen zur gegenseitigen Verbindung im Wesentlichen so, wie sie sind; und das Entfernen des Ofens (9) und das Ersetzen desselben durch einen Wärmetauscher (90), der mit heißem Fluid versorgt wird; und
- das Zuführen von Xylose in den Eingang der Versorgungspumpe (2).

**2.** Das Verfahren gemäß einem oder mehreren der obigen Ansprüche, **dadurch gekennzeichnet, dass** es auch die Wiederverwendung der Abtriebssäule (15) vorsieht.

**3.** Das Verfahren gemäß einem oder mehreren der obigen Ansprüche, **dadurch gekennzeichnet, dass** es die Installation mindestens eines zusätzlichen Reaktors (7a), parallel zu dem mindestens einen Reaktor (7) und so, dass der Reaktor (7) und der zusätzliche Reaktor (7a) wahlweise verwendet werden können, in dem reaktiven Abschnitt vorsieht.

**4.** Das Verfahren gemäß einem oder mehreren der obigen Ansprüche, **dadurch gekennzeichnet, dass** der mindestens eine zusätzliche Reaktor (7a) von der Wiederverwendung eines vorhandenen Reaktors oder Gefäßes stammt, der/das von einer stillgelegten Raffinerie stammt oder zuvor für andere Funktionen verwendet wurde.

**Revendications**

**1.** Procédé de récupération d'une raffinerie de pétrole désaffectée pour obtenir une usine adaptée pour produire du xylitol à partir de xylose, ladite raffinerie comprenant au moins une usine pour l'hydrogénation catalytique de produits pétroliers (1) pourvue au moins des composants suivants :

- au moins une pompe d'alimentation (2) ;
- un moyen pour introduire de l'hydrogène ;
- un moyen d'échange de chaleur comprenant au moins une unité de préchauffage et au moins un four (9) ;
- au moins une section à réaction qui comprend au moins un réacteur (7) ;
- un moyen de séparation gaz/liquide ;
- au moins une colonne (15) pour rectifier le produit ayant subi la réaction ;
ledit procédé étant **caractérisé en ce qu'**il comprend une étape de réutilisation au moins partielle desdits composants afin d'obtenir une usine permettant l'hydrogénation continue du xylose pour obtenir du xylitol (100), et **en ce qu'**il comprend les étapes suivantes :

- supprimer, dans ladite section à réaction, le catalyseur qui est présent et le remplacer par un catalyseur qui est adapté pour faciliter la réaction d'hydrogénation du xylose pour obtenir du xylitol ;
- conserver substantiellement tels quels au moins ladite au moins une pompe d'alimentation (2), ledit moyen d'introduction d'hydrogène, ladite au moins une unité de préchauffage, ledit moyen de séparation gaz/liquide et les conduites d'interconnexion mutuelle correspondantes, et retirer ledit four (9) et le remplacer par un échangeur de chaleur (90) alimenté par un fluide chaud ; et
- introduire du xylose en entrée de ladite pompe d'alimentation (2).

**2.** Procédé selon l'une ou plusieurs des revendications précédentes, **caractérisé en ce qu'**il prévoit également la réutilisation de ladite colonne d'extraction (15).

**3.** Procédé selon l'une ou plusieurs des revendications précédentes, **caractérisé en ce qu'**il comprend l'installation, dans ladite section à réaction, d'au moins un réacteur additionnel (7a) en parallèle audit au moins un réacteur (7) et de manière telle que ledit réacteur (7) et ledit réacteur additionnel (7a) peuvent être utilisés de manière sélective.

**4.** Procédé selon l'une ou plusieurs des revendications précédentes, **caractérisé en ce que** ledit au moins un réacteur additionnel (7a) est obtenu par la réutilisation d'un réacteur ou d'un réservoir existant qui provient d'une raffinerie désaffectée ou qui était précédemment utilisé pour d'autres fonctions.

Fig. 1

*Fig.2*

EP 3 476 918 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015181279 A

- WO 2014033762 A